# EUROPÄISCHE PATENTSCHRIFT

(11) **EP 2 486 144 B1**
(45) Veröffentlichungstag und Bekanntmachung des Hinweises auf die Patenterteilung: **16.10.2013**
(21) Anmeldenummer: 10732641.5
(22) Anmeldetag: 16.06.2010
(51) Int. Cl.: C12Q 1/48, C12Q 1/56

(54) **VERFAHREN ZUR BESTIMMUNG VON FAKTOR XIII MITTELS NAD(P)H-ANALOGA**
METHOD OF DETERMINING FACTOR XIII BY MEANS OF NAD(P)H-ANALOGUES
PROCÉDÉ DE DÉTERMINATION DU FACTEUR XIII AU MOYEN D'ANALOGUES DE NAD(P)H

(30) Priorität: 05.10.2009 DE 102009048198
(43) Veröffentlichungstag der Anmeldung: 15.08.2012
(73) Patentinhaber: Siemens Healthcare Diagnostics Products GmbH, 35041 Marburg (DE)
(72) Erfinder: CHRIST, Gerlinde, 35043 Marburg (DE); KAPPEL, Andreas, 61462 Koenigstein (DE); VITZTHUM, Frank, 35094 Lahntal (DE)
(86) Internationale Anmeldenummer: PCT/EP2010/003611
(87) Internationale Veröffentlichungsnummer: WO 2011/042071

(56) Entgegenhaltungen:
- EP-A1- 0 632 270
- EP-A2- 0 336 353
- WO-A1-90/04038
- CN-A- 101 477 129
- US-A- 5 015 588
- US-B2- 7 195 891
- KARPATI L ET AL: "A modified, optimized kinetic photometric assay for the determination of blood coagulation factor XIII activity in plasma" CLINICAL CHEMISTRY, AMERICAN ASSOCIATION FOR CLINICAL CHEMISTRY, WASHINGTON, DC, Bd. 46, Nr. 12, 1. Januar 2000 (2000-01-01), Seiten 1946-1955, XP002317468 ISSN: 0009-9147
- FICKENSCHER K ET AL: "A PHOTOMETRIC ASSAY FOR BLOOD COAGULATION FACTOR XIII" THROMBOSIS AND HAEMOSTASIS, SCHATTAUER GMBH, DE; US, Bd. 65, Nr. 5, 6. Mai 1991 (1991-05-06), Seiten 535-540, XP009048407 ISSN: 0340-6245 in der Anmeldung erwähnt
- MUSZBEK L ET AL: "KINETIC DETERMINATION OF BLOOD COAGULATION FACTOR XIII IN PLASMA" CLINICAL CHEMISTRY, AMERICAN ASSOCIATION FOR CLINICAL CHEMISTRY, WASHINGTON, DC, Bd. 31, Nr. 1, 1. Januar 1985 (1985-01-01) , Seiten 35-40, XP002036558 ISSN: 0009-9147 in der Anmeldung erwähnt
- Kappel A et al: "Quantification of coagulation factor XIII activity by a thio-NADH based assay using factor XIII immuno-depleted plasma as a diluent for calibration" Clinical Chemistry and Laboratory Medicine 10. September 2010 (2010-09-10), XP002603617 DOI: 10.1515/CCLM.2010.339 Gefunden im Internet: URL:http://www.reference-global.com/doi/ab s/10.1515/CCLM.2010.339 [gefunden am 2010-10-05]

## Beschreibung

Die vorliegende Erfindung liegt auf dem Gebiet der in vitro-Diagnostik und betrifft ein Verfahren zur Bestimmung des Blutgerinnungsfaktors XIII (Faktor XIII, F XIII) sowie ein Testkit zur Durchführung des Verfahrens.

Faktor XIII ist ein Blutgerinnungsfaktor, der am Ende der Blutgerinnungskaskade wirkt und eine wichtige Rolle für den dauerhaften Wundverschluss spielt. In der letzten Phase der Blutgerinnung, der Fibrinbildung, spaltet Thrombin Fibrinogen. Die so entstehenden Fibrinmonomere aggregieren spontan zu langen Fasern und schließlich zu einem dichten, verzweigten Netz aus löslichen Fibrinpolymeren. Auch Faktor XIII wird durch Thrombin aktiviert, wodurch Faktor XIII entsteht. Faktor XIIIa bewirkt eine Quervernetzung der Fibrinpolymere, wodurch das Fibringerinnsel mechanisch stabiler, weniger deformierbar und resistenter gegen Auflösung durch Plasmin wird. Ein kongenitaler oder erworbener Faktor XIII-Mangel kann zu Blutungsneigung, Wundheilungsstörungen sowie zu Aborten führen. Aufgrund der klinischen Relevanz ist die Bestimmung von Faktor XIII zum Ausschluss oder zur Feststellung eines Faktor XIII-Mangels ein wichtiger Bestandteil der Gerinnungsdiagnostik.

Faktor XIIIa, die aktivierte Form des katalytisch inaktiven Proenzyms Faktor XIII, ist eine Transglutaminase, die die dreidimensionale Quervernetzung der Fibrinpolymere durch Ausbildung intermolekularer Amidbindungen zwischen Lysyl- und Glutaminyl-Aminosäureseitenketten der Fibrinmoleküle katalysiert. Bei dieser Reaktion entsteht Ammoniak (NH₃) bzw. Ammoniumionen (NH₄⁺) werden freigesetzt. Dieses Phänomen wird in verschiedenen Testverfahren zur Bestimmung von Faktor XIII ausgenutzt: Im Folgenden wird der Begriff Ammoniak synonym für Ammoniak und Ammoniumionen verwendet.

Muszbek, L. et al. [Clin. Chem. (1985) 31(1), 35-40] beschreiben ein Verfahren zur Bestimmung von Faktor XIII in defibrinierten Plasmaproben, wobei der Faktor XIII der Probe mit Thrombin zu Faktor XIIIa aktiviert wird. Ferner wird die Probe mit β-Casein und Ethylamin vermischt, die als Substrate für die Ausbildung intermolekularer Amidbindungen durch Faktor XIIIa dienen. Um den bei dieser Reaktion freigesetzten Ammoniak quantitativ nachzuweisen, wird die Probe zusätzlich mit NADPH (Nicotinsäureamid-Adenin-Dinukleotid-Phosphat-Hydrid) und mit Komponenten einer NADPH-abhängigen Indikatorreaktion vermischt, nämlich mit Glutamatdehydrogenase (GLDH) und α-Ketoglutarat. In Anwesenheit von Ammoniak wandelt GLDH α-Ketoglutarat in Glutamat um. Diese Reaktion verbraucht zusätzlich NADPH, und es entsteht NADP+ (Nicotinsäureamid-Adenin-Dinukleotid-Phosphat), die oxidierte Form von NADPH. NADP+ hat ein anderes Absorptionsspektrum als NADPH, so dass sich die Absorption (auch Extinktion oder optische Dichte genannt) des Testansatzes proportional zum NADPH-Verbrauch und damit proportional zur Ammmoniakmenge und damit proportional zur Faktor XIII-Menge bzw. -Aktivität ändert. Alternativ kann in diesem Testansatz NADH anstatt NADPH verwendet werden. Im Gegensatz zu NAD(P)+ besitzt NAD(P)H neben einem Absorptionsmaximum bei circa 260 nm ein Absorptionsmaximum bei circa 340 nm. Die exakte Lage von Absorptionsmaxima hängt in der Regel von verschiedenen Parametern ab, insbesondere von der Dielektrizitätskonstanten und dem pH-Wert der Lösung. Im Allgemeinen liegt das Absorptionsmaximum des NAD(P)H im Bereich von 335 bis 345 nm. Die Messung der Änderung der Absorption des Testansatzes bei einer Wellenlänge von 340 nm ermöglicht die quantitative Bestimmung des Faktors XIII in einer Probe.

EP 336 353 A2 bzw. Fickenscher et al. (Thromb Haemost. 1991, 65(5): 535-40) beschreiben ein ähnliches Verfahren, mit dem Faktor XIII über das freigesetzte Ammoniak quantifiziert wird. EP 336 353 A2 beschreibt ein Verfahren zur Bestimmung von Faktor XIII in unvorbehandelten, fibrinhaltigen Plasmaproben. Um die Entstehung störender Fibringerinnsel im Reaktionsansatz zu unterbinden, wird die Probe zusätzlich mit einem Fibrinaggregationshemmer vermischt. Der Faktor XIII der Probe wird mit Thrombin in Gegenwart von Ca²⁺-Ionen zu Faktor XIIIa aktiviert. Ferner wird die Probe mit einem synthetischen, glutaminhaltigen Peptid und Glycinethylester vermischt, die als Substrate für die Ausbildung intermolekularer Amidbindungen durch Faktor Xllla dienen. Um den bei dieser Reaktion freigesetzten Ammoniak quantitativ nachzuweisen, wird die Probe zusätzlich mit NADH (Nicotinsäureamid-Adenin-Dinukleotid-Hydrid) und mit Komponenten einer NADH-abhängigen Indikatorreaktion vermischt, nämlich mit Glutamatdehydrogenase (GLDH) und α-Ketoglutarat. In Anwesenheit von Ammoniak wandelt GLDH α-Ketoglutarat in Glutamat um. Diese Reaktion verbraucht zusätzlich NADH, und es entsteht NAD+, die oxidierte Form von NADH. NAD+ hat ein anderes Absorptionsspektrum als NADH, so dass sich die Absorption des Testansatzes proportional zum NADH-Verbrauch und damit proportional zur Ammmoniakmenge und damit proportional zur Faktor XIII-Menge bzw. -Aktivität ändert. Alternativ kann in diesem Testansatz NADPH anstatt NADH verwendet werden. Die Messung der Änderung der Absorption des Testansatzes bei einer Wellenlänge von 340 nm ermöglicht die quantitative Bestimmung des Faktors XIII in einer Probe. Ein kommerzieller Test, der auf dem in EP 336 353 A2 beschriebenen Testprinzip beruht, ist der Berichrom® F XIII Test von Siemens Healthcare Diagnostics.

Die beschriebenen Verfahren haben den Nachteil, dass sie relativ anfällig gegenüber probenintrinsischen Störsubstanzen sind. Patientenproben können in Einzelfällen abnormal hohe Konzentrationen einer oder mehrerer intrinsischer, also körpereigener Substanzen enthalten, die sich bei Überschreitung einer tolerablen Konzentration in photometrischen Detektionsverfahren als störend erweisen und sich zu einem systematischen Fehler auswirken können. Probleme bereiten bekanntermaßen hämolytische, ikterische und/oder lipämische Serum- oder Plasmaproben, sogenannte HIL-Proben, die über abnormal hohe Hämoglobin-, Bilirubin- und/oder Triglyzerid-Konzentrationen verfügen. Abnormal hohe Konzentrationen dieser interferierenden Substanzen können durch einen pathologischen Zustand des Patienten oder aber durch eine unsachgemäße Probengewinnung oder -lagerung verursacht werden.

Der vorliegenden Erfindung lag damit die Aufgabe zugrunde, ein Verfahren zur Bestimmung von Faktor XIII bereit zu stellen, das weniger störanfällig gegenüber probenintrinsischen Störsubstanzen ist. Im Besonderen bestand die Aufgabe darin, ein Verfahren bereit zu stellen, das die Bestimmung von Faktor XIII in Proben mit hohen Hämoglobin-, Bilirubin- und/oder Triglyzerid-Konzentrationen ermöglicht.

Die Aufgabe wird dadurch gelöst, dass ein bekanntes Verfahren zur Bestimmung von Faktor XIII, bei dem
a) die Probe mit I. einer Substanz zur Aktivierung des Faktor XIII zu Faktor XIIIa (z. B. mit Thrombin in Gegenwart von Ca²⁺-Ionen), II. mit einem Akzeptorsubstrat für Faktor Xllla (z. B. mit einem glutaminhaltigen Peptid), III. mit einem Aminogruppendonorsubstrat für Faktor XIIIa (z. B. mit einem primären Amin), IV. mit NADH oder NADPH und V. mit einem ein Mittel, das in Gegenwart von Ammoniak NADH zu NAD+ zu oxidieren vermag (z. B. bestehend aus Glutamatdehydrogenase und αKetoglutarat), vermischt wird und
b) die Änderung der Absorption des Testansatzes gemessen wird,
insofern verändert wird, dass anstelle von NADH oder NADPH ein Analogon von NADH oder NADPH, ein sogenanntes NAD(P)H-Analogon eingesetzt wird, das ein Absorptionsmaximum aufweist, das oberhalb von 350 nm liegt, wobei das NAD(P)H-Analogon Thio-NAD(P)H oder Seleno-NAD(P)H ist.

Zur Vereinfachung wird der Terminus NAD(P)H verwendet, wenn Ausführungen sowohl die phosphorylierte als auch die nicht-phosphorylierte Form von NADH betreffen, also wenn NADH und NADPH gleichermaßen gemeint sind. Der Terminus NAD(P)+ wird verwendet, wenn Ausführungen sowohl die phosphorylierte als auch die nicht-phosphorylierte Form von NADH im oxidierten Zustand betreffen, also wenn NAD+ und NADP+ gleichermaßen gemeint sind.

Unter dem Begriff "NAD(P)H-Analogon" ist im Sinne der vorliegenden Erfindung eine Substanz zu verstehen, die wie NAD(P)H als Kosubstrat für eine NAD(P)H-abhängige Dehydrogenase, die Ammoniak umsetzt, fungieren kann. Erfindungsgemäß muss es sich um ein Analogon handeln, bei dem die oxidierte und reduzierte Form unterschiedliche Absorptionsmaxima haben, wobei das Absorptionsmaximum der reduzierten Form des NAD(P)H-Analogons oberhalb von 350 nm liegt.

Bei den NAD(P)H-Analoga handelt es sich vorzugsweise um organische, zyklische und heterozyklische Verbindungen, die eine Redoxreaktion basierend auf dem Konzept des Transfers eines Hydrid-Ions erlauben, d. h. eine Redoxreaktion, bei der das Äquivalent eines Protons mit zwei Elektronen übertragen wird.

Insbesondere sind hier organische Verbindungen, vorzugsweise heterozyklische Verbindungen eingeschlossen, die von einer chinoiden Form bei Reduktion, d. h. Aufnahme eines Hydrid-Ions, in eine benzoide Form und umgekehrt überführt werden können. Bevorzugt sind insbesondere Abkömmlinge des Pyridins. Hierbei sind Strukturanaloga des Nikotinamids bevorzugt.

Es gibt NAD(P)H-Strukturanaloga, bei denen die Nikotinamidgruppe des NAD(P)H gegen eine andere Gruppe ausgetauscht wurde. Bevorzugt sind hier organische, zyklische und heterozyklische Verbindungen, die eine Redoxreaktion basierend auf dem Konzept des Transfers eines Hydrid-Ions erlauben, d.h. eine Redoxreaktion, bei der das Äquivalent eines Protons mit zwei Elektronen übertragen wird. Insbesondere sind hier organische Verbindungen, vorzugsweise heterozyklische Verbindungen eingeschlossen, die von einer chinoiden Form bei Reduktion, d.h. Aufnahme eines Hydrid-Ions, in eine benzoide Form und vice versa überführt werden können. Bevorzugt sind insbesondere Abkömmlinge des Pyridins.

Bei den Pyridinabkömmlingen sind Analoga für die Verwendung in dem erfindungsgemäßen Verfahren auszuschließen, die mit einem Substituenten in Position 4 des Pyridinringes ausgestattet sind, z. B. 4-Methylnikotinamid-Adenin-Dinukleotid. Derartige Analoga können zwar chemisch, beispielsweise durch Dithionit reduziert werden (M. Jarman and F. Searle: Potential coenzyme inhibitors V - The synthesis and some protperties of 4-methylnicotinamide adenine dinucleotide, Biochemical Pharmacology, Vol. 21, 455-464, 1972), sind aber aus sterischen Gründen kaum für einen enzymkatalysierten Transfer von Hydrid-Ionen geeignet.

Es gibt auch NAD(P)H-Strukturanaloga, bei denen die Pyridingruppe in Position 3 eine Seitenkette trägt. Als Substituenten kommen vorzugsweise Carbonylverbindungen in Frage, beispielsweise eine Aldehyd-, Acetyl-, Carbonsäure-, Thioaldehyd-, Thioacetyl-, Thiocarbonsäure-, Thiocarbonsäureamid-, Selenoaldehyd-, Selenoacetyl-, Selenocarbonsäure-, Selenocarbonsäureamidgruppe, etc.

Es gibt ferner NAD(P)H-Strukturanaloga, mit den Pyridinanaloga 3-Acetylpyridin, 3-(Carb)-Aldehydpyridine, Thionikotinamid und Selenonikotinamid. Aus dieser Gruppe ist das Thionikotinamid besonders bevorzugt.

Die Absorptionsmaxima von Seleno-NAD(P)H, Thio-NAD(P)H, 3-Acetylpyridin-Adenosindinukleotidhydrid und 3-Aldehydpyridin-Adenosindinukleotidhydrid liegen so, dass die Reaktion beispielsweise jeweils bei circa 417 nm, 400 nm, 365 nm und 358 nm verfolgt werden können (Werner Hensel, Dagmar Rakow, Wolfram Christ: Convenient method for preparation and purification of nicotinamide mononucleotide analogs. Analytical Biochemistry, 68, 128-137, 1975; Christ, W. and Coper H.: Properties of selenonictoinamide-adenine dinucleotide phosphate, an analogue of NADP. FEBS Letters, Vol. 2, Number 4, 267-269).

Das Dokument US 7,195,891 B2 beschreibt, dass in einem spektrometrischen Verfahren zum Nachweis von Myeloperoxidase (einem Infektions- bzw. Entzündungsmarker) NADH, NADPH, Thio-NADH, Thio-NADPH, Acetyl-NADH oder Acetyl-NADPH (R)-Laktat gleichermaßen als Elektronenakzeptoren verwendet werden können.

Das Dokument CN-A-101477129 beschreibt ein spezifisches, sensitives und wenig fehleranfälliges Nachweisverfahren, bei dem u.a. die oxidierten Substanzen NAD⁺, NADP⁺ und/oder Thio-NAD⁺ eine Verwendung finden.

NAD(P)H-Analoga können andere enzymkinetische Eigenschaften als NAD(P)H aufweisen. Beispielsweise können Analoga schlechter umgesetzt werden. Dies kann durch Anpassung der Konzentration des Analogons, des Enzyms oder anderer Komponenten im Reaktionsansatz zumindest teilweise so ausgeglichen werden, dass die Reaktion noch in ausreichendem Maße abläuft. Ein Analogon, dass von einem entsprechenden Enzym nicht umgesetzt werden kann oder gar als Inhibitor auftritt, ist nicht erfindungsgemäß. Beispielsweise ist davon auszugehen, dass Pyridin-Analoga mit einem Substituenten in Position 4, z.B. 4-Methylnikotinamid-Adenine-Dinukleotid nicht geeignet sind (siehe oben).

Die Suche nach bzw. das Auffinden von NAD(P)H-Analoga kann mit Hilfe von Enzymreaktionen, die von NAD(P)H-abhängigen Dehydrogenasen katalysiert werden, erfolgen. Beispielsweise können bei einem entsprechenden Screeningverfahren die Systeme verwendet werden, die auch bei einem F XIII-Test eingesetzt werden. Zur Identifizierung von NAD(P)H-Analoga, die zur Verwendung in dem erfindungsgemäßen Verfahren geeignet sind, werden bevorzugterweise zunächst deren spektrale Eigenschaften bestimmt. Vorzugsweise werden die spektralen Eigenschaften der reduzierten Form ermittelt. Ein Analogon muss ein Absorptionsmaximum oberhalb von 350 nm aufweisen. Das entsprechende Analogon wird zu einer Testlösung gegeben und die Veränderung der Absorption in dem relevanten Wellenlängenbereich verfolgt. Vorzugsweise wird das Analogon hierbei in unterschiedlichen Konzentrationen eingesetzt. Beispielsweise können hierzu 20 µl einer Lösung der reduzierten Form eines Analogons mit unterschiedlichen Konzentrationen in 2880 µl einer Testlösung pH 7,9 pipettiert werden, die 84,7 mM Imidazolpuffer, 13,6 mM 2-Oxoglutarat, 217 mM Ammoniumacetat, 0,9 mM Ethylendiamintetraacetat und 1,7 mM Adenosin-5'-Diphosphat enthält. Wird das reduzierte Analogon umgesetzt, d. h. oxidiert, erfolgt eine Abnahme der Absorption, die photometrisch verfolgt werden kann.

Die Synthese von NAD(P)H-Analoga kann über die Pyridinanaloga erfolgen, die zunächst chemisch synthetisiert werden, ebenfalls mittels chemischer Verfahren zu den Mononukleotidanaloga alkyliert werden, um dann beispielsweise enzymatisch mit Hilfe einer NAD-Pyrophosphorylase oder chemisch mit Adenosintriphosphat (ATP) oder Adenosindiphosphat (ADP) zum entsprechenden NAD(P)+ bzw. NAD(P)H-Analogon kondensiert werden. In der Regel wird so zunächst die oxidierte Form, d.h. das NAD(P)+-Analogon synthetisiert. Die Reduktion des Diphosphopyridinnukleotids bzw. des NAD(P)+-Analogons kann dann beispielsweise durch Dithionit erfolgen (Werner Hensel, Dagmar Rakow, Wolfram Christ: Convenient method for preparation and purification of nicotinamide mononucleotide analogs, Analytical Biochemistry, 68, 128-137, 1975; M. Jarman and F. Searle: Potential coenzyme inhibitors V - The synthesis and some protperties of 4-methylnicotinamide adenine dinucleotide, Biochemical Pharmacology, Vol. 21, 455-464, 1972).

Da Thio-NAD(P)H ein Absorptionsmaximum aufzeigt, das im Vergleich zu anderen Analoga ganz besonders im längerwelligen Bereich liegt, zudem enzymatisch von den meisten Dehydrogenasen gut umgesetzt wird, vergleichsweise stabil und kommerziell verfügbar ist, ist dieses NAD(P)H-Analogon in ganz besonderer Weise bevorzugt. Ein weiteres bevorzugtes NAD(P)H-Analogon ist Seleno-NAD(P)H.

Thio-NADH (Thionikotinsäureamid-Adenin-Dinukleotid-Hydrid) bzw. Thio-NADPH (Thionikotinsäureamid-Adenin-Dinukleotidphosphat-Hydrid) können in analoger Weise wie NADH und NADPH oxidiert werden, nämlich zu Thio-NAD+ bzw. Thio-NADP+ und verfügen bekanntermaßen über andere optische Eigenschaften als NADH bzw. NADPH, so dass eine Änderung der Absorption infolge der Oxidation zu Thio-NAD+ bzw. Thio-NADP+ bei Wellenlängen von etwa 340 nm bis etwa 430 nm gemessen werden kann.

Ein Gegenstand der vorliegenden Erfindung ist die Verwendung von Thio-NADH bzw. von Thio-NADPH (also Thio-NAD(P)H) in einem Verfahren zur Bestimmung von Faktor XIII in einer Probe.

Ein weiterer Gegenstand der vorliegenden Erfindung ist die Verwendung von Seleno-NADH bzw. von Seleno-NADPH (also Seleno-NAD(P)H) in einem Verfahren zur Bestimmung von Faktor XIII in einer Probe.

Die vorliegende Erfindung betrifft ferner ein Verfahren zur Bestimmung von Faktor XIII in einer Probe, bei dem
a) die Probe mit einem oder mehreren Reagenzien enthaltend
   I. eine Substanz oder ein Substanzgemisch zur Aktivierung des Faktor XIII zu Faktor XIIIa,
   II. ein Akzeptorsubstrat für Faktor XIIIa mit mindestens einer Glutaminylgruppe,
   III. ein Aminogruppendonorsubstrat für Faktor XIIIa,
   IV. ein NAD(P)H-Analogon mit einem Absorptionsmaximum das oberhalb von 350 nm liegt, und
   V. ein Mittel, das in Gegenwart von Ammoniak NAD(P)H zu NAD(P)+ oder ein NAD(P)H-Analoga zu dem entsprechenden NAD(P)+-Analoga zu oxidieren vermag,
   vermischt wird und
b) die Änderung der Absorption des Testansatzes gemessen wird
   und wobei das NAD(P)H-Analogon Thio-NAD(P)H oder Seleno-NAD(P)H ist.

Als Substanz zur Aktivierung des Faktor XIII zu Faktor Xllla eignet sich insbesondere Thrombin, beispielsweise humanen oder bovinen Ursprungs oder auch rekombinant hergestelltes Thrombin. Ebenfalls geeignet sind Substanzen oder Substanzgemische wie beispielsweise Faktor Xa, das Schlangengift Ecarin oder eine Mischung aus Tissue factor, Phospholipiden und Ca²⁺-Ionen, die indirekt eine Aktivierung des Faktors XIII bewirken, indem sie das in der Probe enthaltene Prothrombin direkt oder indirekt zu Thrombin aktivieren, welches dann wiederum den Faktor XIII aktiviert.

Unter dem Begriff "Akzeptorsubstrat für Faktor XIIIa mit mindestens einer Glutaminylgruppe" ist ein Polypeptid oder Peptidmimetikum zu verstehen, das mindestens eine Glutaminylgruppe, beispielsweise von der Aminosäure Glutaminsäureamid aufweist. Bekannte Akzeptorsubstrate für Faktor Xllla sind z. B. β-Casein sowie eine Vielzahl synthetischer Peptide. Geeignete synthetische Peptide sind beispielweise in EP 314 023 A2 beschrieben.

Unter dem Begriff "Aminogruppendonorsubstrat für Faktor XIIIa" sind insbesondere primäre Amine zu verstehen. Bevorzugte primäre Amine sind Ethanolamin, Putrescin, Cadaverin, Diaminoethan, Aminoethan. Besonders bevorzugte primäre Amine sind Glycinethylester oder Glycinmethylester.

Ein "Mittel, das in Gegenwart von Ammoniak NADH zu NAD+ zu oxidieren vermag" ist bevorzugterweise ein Enzym/Substratsystem, welches ein Enzym und ein Substrat für das Enzym umfasst, wobei das Enzym katalytisch auf das Substrat einwirkt und dabei in Gegenwart von Ammoniak NADH zu NAD+ bzw. NADPH zu NADP+ (also NAD(P)H zu NAD(P)+) und damit auch Thio-NADH zu Thio-NAD+ bzw. Thio-NADPH zu Thio-NADP+ oder andere erfindungsgemäße NAD(P)H-Analoga oxidiert.

Geeignete Enzym/Substratsysteme sind Dehydrogenasen und deren Substrate, die NAD(P)H bzw. NAD(P)H-Analoga als Cofaktor zusammen mit Ammoniak umsetzen. Hierbei handelt es sich insbesondere um Aminosäuredehydrogenasen, wie D-Aminosäuredehydrogenasen und L-Aminosäuredehydrogenasen. Beispiele hierfür sind die Alanin-, Glutamat-, Serin-2-, Valin-, Leucin-, Glycin-, Lysin-, Tryptophan-, Phenylalanin-, Asparat-, Diaminopimelat-, N-Methylalanin, *L*-*erythro*-3,5-diaminohexanoat-, und die 2,4-Diaminopentanoat-Dehydrogenase. Die jeweiligen Substrate für die beschriebene Reaktionsrichtung der Aminierung sind Ammoniak sowie die respektiven Oxo-Verbindungen. Entsprechend der Reihenfolge der obigen Beispiele sind dies die 2-Oxosäuren für die Aminosäuredehydrogenasen sowie Pyruvat, alpha-Ketoglutarat (2-Oxoglutarat), 3-Hydroxypyruvat, 3-Methyl-2-oxobutanoat, 4-Methyl-2-oxopentanoat, Glyoxylat, 1,2-Didehydropiperidine-2-carboxylat, Indol-3-yl-pyruvat, Phenylpyruvat, Oxaloacetat, L-2-Amino-6-oxoheptanedioat, Pyruvate und Methylamin, (S)-5-Amino-3-oxohexanoate, und L-2-Amino-6-oxoheptanedioate. Geeignete Enzym/Substratsysteme sind also z. B. das Glutamatdehydrogenase/Ketoglutarat-System oder das Alanindehydrogenase/Pyruvat-System oder das Serin-2-dehydrogenase/3-Hydroxypyrovat-System oder das Valindehydrogenase/3-Methyl-2-Oxobutanoat-System oder das Leucindehydrogenase/ 4-Methyl-2-Oxopentanoat-System oder das Glycindehydrogenase/Glyoxylat-System oder das Lysindehydrogenase/1,2-Didehydropiperidin-2-Carboxylat-System oder das Phenylalanin/Phenylpyruvat-System oder das Aspartatdehydrogenase/Oxaloacetat-System oder das Glucose-6-Phosphat-Dehydrogenase/D-Glucono-1,5-lakton-6-Phosphat-System.

In einer bevorzugten Ausführungsform des erfindungsgemäßen Verfahrens wird die Probe, bevorzugterweise eine Plasmaprobe, zusätzlich mit einem Fibrinaggregationshemmer vermischt. Fibrinaggregationshemmer sind Substanzen, die die Aggregation von Thrombin-induzierten Fibrinmonomeren verhindern. Auf diese Weise wird die Entstehung eines Fibringerinnsels in einer fibrinogenhaltigen Probe verhindert, welches ansonsten die Absorptionssmessung des Testansatzes negativ beeinträchtigen würde. Bevorzugte Fibrinaggregationshemmer sind synthetische Peptide, wie z. B. ein Peptid der Sequenz Gly-Pro-Arg-Pro (kommerziell erhältlich als Pefabloc®FG, Pentapharm, Schweiz). Weitere bevorzugte Peptide, die als Fibrinaggregationshemmer verwendet werden können, insbesondere das bevorzugte Peptid der Sequenz Gly-Pro-Arg-Pro-Ala sind in EP 456 152 A2 beschrieben.

In einer weiteren Ausführungsform wird die Probe zusätzlich mit einer Heparinneutralisierenden Substanz vermischt, beispielsweise mit Hexadimethrinbromid, auch bekannt als Polybrene®, um die Thrombin-inhibierende Wirkung von Heparin, das z. B. in Proben von Heparin-therapierten Patienten vorhanden sein kann, auszuschalten.

Die Komponenten I bis V, die mit der Probe zu einem Testansatz vermischt werden, können jeweils einzeln, d. h. in Form einzelner Reagenzien und nacheinander mit der Probe vermischt werden; sie können aber auch in einem einzigen Reagenz vereinigt sein, welches in einem einzigen Pipettierschritt mit der Probe vermischt wird. Das Reagenz oder die Reagenzien umfassen vorzugsweise eine Puffermatrix, in der die Substanzen gelöst sind. Eine geeignete Puffermatrix enthält z. B. HEPES, Bicin, NaCl, Albumin und/oder Konservierungsmittel, wie z. B. Natriumazid und hat vorzugsweise einen pH-Wert von 6.0 bis 9.0, besonders bevorzugt von 6.5 bis 8.5. Da Calciumionen für die Aktivierung von FXIII notwendig sind, enthält die Puffermatrix ferner ein Calciumsalz, bevorzugterweise Calciumchlorid. Das Vermischen des Reagenzes oder der Reagenzien mit der Probe kann manuell oder an automatischen Gerinnungsmessgeräten durchgeführt werden.

Die Menge des NAD(P)H-Analogons, die dem Testansatz zugegeben wird, ist je nachdem welches Mittel, das in Gegenwart von Ammoniak das NAD(P)H-Analogon zum entsprechend NADP+-Analogon zu oxidieren vermag, verwendet wird, zu optimieren. Bei Verwendung des Glutamatdehydrogenase/Ketoglutarat-Systems ist sie beispielsweise für Thio-NADH vorzugsweise so gewählt, dass die Endkonzentration des Thio-NADH im Testansatz 10 - 500 µM, bevorzugterweise 50 - 400 µM beträgt.

Sofern das Glutamatdehydrogenase/Ketoglutarat-System als Mittel, das in Gegenwart von Ammoniak NADH zu NAD zu oxidieren vermag, in dem erfindungsgemäßen Verfahren eingesetzt wird, ist die Menge an Glutamatdehydrogenase, die dem Testansatz zugegeben wird, so gewählt, dass die Endkonzentration im Testansatz 2 - 500 IU/mL, bevorzugterweise 5 - 250 IU/mL beträgt.

Als Probenmaterial eignet sich insbesondere Fibrinogen-haltiges Plasma. Aber auch in defibriniertem Plasma kann Faktor XIII gemäß dem erfindungsgemäßen Verfahren bestimmt werden.

Die Messung der Absorptionssänderung (ΔE) des Testansatzes erfolgt mit Hilfe eines Photometers umfassend eine Lichtquelle, die einen Lichtstrahl durch den zu messenden Testansatz sendet und einen Detektor, der die Intensität des durchgetretenen Lichts misst und in ein elektrisches Signal umwandelt. Die Messung der Absorptionsänderung erfolgt mit Licht einer Wellenlänge von etwa 340 nm bis etwa 430 nm, bevorzugterweise mit Licht einer Wellenlänge von etwa 380 bis etwa 420 nm, ganz besonders bevorzugt mit Licht einer Wellenlänge von etwa 390 bis etwa 410 nm. Die sich pro Zeiteinheit ändernde Absorption korreliert mit der Faktor XIII-Aktivität. Die Abnahme der Absorption (E) des Testansatzes durch den Thio-NADH- bzw. Thio-NADPH-Verbrauch ist insbesondere im linearen Bereich der Reaktionskinetik direkt proportional zur Faktor XIII-Aktivität. Die Faktor XIII-Aktivität einer Probe wird bevorzugterweise durch den Vergleich mit einer Normalplasmapoolprobe, die per definitionem eine Faktor XIII-Aktivität von 100 % aufweist, berechnet.

Ein weiterer Gegenstand der vorliegenden Erfindung ist ein Testkit zur Durchführung des erfindungsgemäßen Verfahrens zur Bestimmung von Faktor XIII in einer Probe, wobei das Testkit folgende Komponenten umfasst:
1. ein erstes Reagenz enthaltend eine Substanz oder ein Substanzgemisch zur Aktivierung des Faktor XIII zu Faktor XIIIa, bevorzugterweise Thrombin;
2. ein zweites Reagenz enthaltend
   - mindestens ein Akzeptorsubstrat mit mindestens einer Glutaminylgruppe für Faktor Xllla, bevorzugterweise ein synthetisches Peptid, das mindestens einen Glutaminylrest als Amin-Akzeptor aufweist,
   - mindestens ein Aminogruppendonorsubstrat für Faktor XIIIa, bevorzugterweise ein primäres Amin, und
   - mindestens ein Mittel, das in Gegenwart von Ammoniak NAD(P)H zu NAD(P)+ oder ein NAD(P)H-Analoga zu dem entsprechenden NAD(P)+-Analoga zu oxidieren vermag, wobei das Mittel bevorzugterweise aus Glutamat-dehydrogenase und Ketoglutarat besteht; und
3. ein drittes Reagenz enthaltend mindestens ein NAD(P)H-Analogon mit einem Absorptionsmaximum das oberhalb von 350 nm liegt, und wobei das NAD(P)H-Analogon Thio-NAD(P)H oder Seleno-NAD(P)H ist.

Die Reagenzien können zusätzlich Konservierungsmittel, Salze, Puffersubstanzen und/oder Stabilisatoren enthalten, wie bespielsweise Natriumazid und Albumin. Die Reagenzien können entweder als Flüssigreagenzien oder als Lyophilisate bereitgestellt werden. Für den Fall, dass einige oder alle Reagenzien des Testkits als Lyophilisate vorliegen, kann das Testkit zusätzlich die zur Lösung der Lyophilisate erforderlichen Lösemittel enthalten, wie z. B. destilliertes Wasser und/oder geeignete Puffer.

In einem bevorzugten Testkit enthält das erste Reagenz, welches Thrombin zur Aktivierung des Faktor XIII zu Faktor XIIIa enthält, zusätzlich Calciumchlorid und/oder einen Fibrinaggregationshemmer und/oder Hexadimethrinbromid.

### Figurenbeschreibung

**Figur 1**
   Faktor XIII-Bestimmung mit Thio-NADH. Die Absorptionsabnahme des Testansatzes verhält sich proportional zur Faktor XIII-Konzentration in der Probe (siehe Beispiel 1).
**Figur 2**
   Faktor XIII-Bestimmung in Hämoglobin-gespikten Plasmaproben (siehe Beispiel 2). Die obere Abbildung zeigt die Ergebnisse des NADH F XIII-Tests. Die untere Abbildung zeigt die Ergebnisse des erfindungsgemäßen Thio-NADH F XIII-Tests. Die gestrichelten horizontalen Linien markieren die 10 %ige Abweichung vom Ausgangswert bei 0 mg/mL Hämoglobin. Der Thio-NADH F XIII-Test überschreitet die 10 %-Grenze erst bei wesentlich höheren Hämoglobin-Konzentrationen als der bekannte NADH F XIII-Test.
**Figur 3**
   Faktor XIII-Bestimmung in Cholesterol-gespikten Plasmaproben (siehe Beispiel 2). Die obere Abbildung zeigt die Ergebnisse des NADH F XIII-Tests. Die untere Abbildung zeigt die Ergebnisse des erfindungsgemäßen Thio-NADH F XIII-Tests. Die gestrichelten horizontalen Linien markieren die 10 %ige Abweichung vom Ausgangswert bei 0 mg/mL Cholesterol. Im Gegensatz zu dem bekannten NADH F XIII-Test überschreitet der erfindungsgemäße Thio-NADH F XIII-Test die 10 %-Grenze gar nicht bei den hier getesteten Cholesterol-Konzentrationen.
**Figur 4**
   Faktor XIII-Bestimmung in Bilirubin-gespikten Plasmaproben (siehe Beispiel 2). Die obere Abbildung zeigt die Ergebnisse des NADH F XIII-Tests. Die untere Abbildung zeigt die Ergebnisse des erfindungsgemäßen Thio-NADH F XIII-Tests. Die gestrichelten horizontalen Linien markieren die 10 %ige Abweichung vom Ausgangswert bei 0 mg/mL Bilirubin. Im Gegensatz zu dem bekannten NADH F XIII-Test überschreitet der erfindungsgemäße Thio-NADH F XIII-Test die 10 %-Grenze gar nicht bei den hier getesteten Bilirubin-Konzentrationen.

### Beispiele

### Beispiel 1:

### Erfindungsgemäße Bestimmung von Faktor XIII mittels Thio-NADH

Es wurden folgende Reagenzien hergestellt:

### Aktivatorreagenz (pH 8.3):

- 292 µM Thio-NADH (Oriental Yeast Company, Rotterdam, Niederlande)
- Thrombin vom Rind (10 IU/mL)
- Gly-Pro-Arg-Pro-Ala-amid als Fibrinaggregationsinhibitor (2g/L)
- Calciumchlorid (1,2 g/L)
- Hexadimethrinbromid (10 mg/L)
- Albumin vom Rind
- Bicin-Puffer (100 mmol/L)

### Nachweisreagenz (pH 6.5):

- Glutamatdehydrogenase 260 (IU/mL)
- Leu-Gly-Pro-Gly-Gln-Ser-Lys-Val-Ile-Gly-amid als F XIII Akzeptorsubstrat (2,4 g/L)
- ADP
- Glycinethylester (1,4 g/L)
- α-Ketoglutarat (2,7 g/L)
- Albumin vom Rind
- HEPES-Puffer (10 mmol/L)

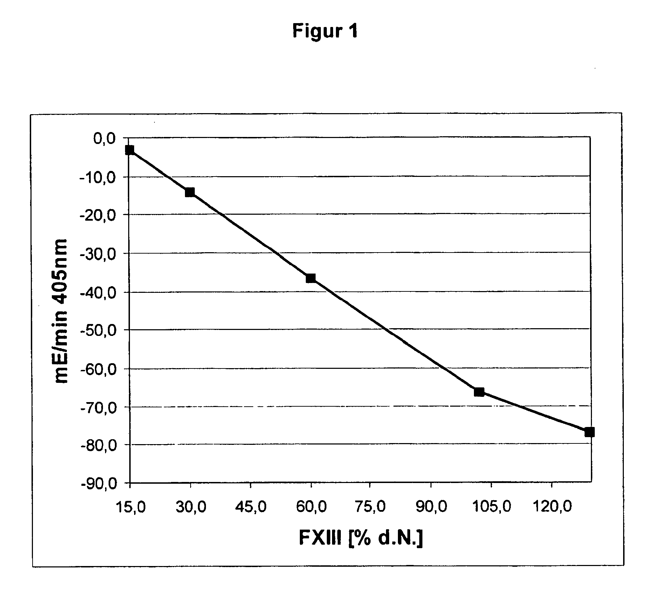

Für den Test wurden auf dem BCS®-XP Gerinnungsanalyzer (Siemens Healthcare Diagnostics Products GmbH, Marburg, Deutschland) 75 µL Aktivator-Reagenz, 75 µL Nachweis-Reagenz und 15 µL Probe in einer Küvette vereint und bei 37 °C inkubiert. Nach 5 Minuten wurde die Messung der Absorption bei einer Wellenlänge von 405 nm gestartet. Zur Auswertung wurde die Änderung der Absorption pro Minute bei 405 nm in einem Zeitfenster von 60 Sekunden - 350 Sekunden nach dem Start der Messung berechnet. Zur Kalibration wurde Standard Humanplasma (Siemens Healthcare Diagnostics Products GmbH, Marburg, Deutschland) als Standard mit einer Faktor XIII-Konzentration von 102 % d.N. (Prozent der Norm) verwendet. Kalibrationspunkte mit niedrigerer Faktor XIII-Konzentration wurden durch Verdünnung des Standards in 0,9 % NaCl-Lösung, Kalibrationspunkte mit höherer Faktor XIII-Konzentration durch ein erhöhtes Volumen des Standards im Test erhalten. Figur 1 zeigt eine typische Kalibrationskurve.

### Beispiel 2:

### Erfindungsgemäße Bestimmung von Faktor XIII mittels Thio-NADH in HIL-Proben

Standard Humanplasma (SHP, Siemens Healthcare Diagnostics) wurde mit steigenden Konzentrationen Hämoglobin, Bilirubin, Triglyceriden oder Cholesterol vermischt, die jeweils in einem konstanten Volumen dem Plasma zudosiert wurden. Dazu wurden 540 µL SHP mit 60 µL einer Lösung der jeweiligen Substanz in verschiedenen Konzentrationen versetzt. Hämoglobin wurde dazu in Tris-gepufferter Saline (TBS: 150 mM NaCl, 50 mM Tris, pH 7.6), Cholesterol in TBS mit 40 mg/mL Humanserumalbumin, und Bilirubin in 0,05 M NaOH gelöst und dem SHP zudosiert. Für die Bestimmung des Ausgangswertes wurden 540 µL SHP mit 60 µL der jeweiligen Pufferlösung ohne zugesetzte Substanz versetzt. Anschließend wurde die Faktor XIII-Aktivität der so gespikten Plasmaproben mit Hilfe des erfindungsgemäßen Verfahrens mittels Thio-NADH (siehe Beispiel 1) in Dreifachbestimmung ermittelt. Zum Vergleich wurden dieselben Proben mit Hilfe des Berichrom® FXIII-Tests (Siemens Healthcare Diagnostics Products GmbH, Marburg, Deutschland), bei dem NADH verwendet wird, gemäß den Herstellerangaben in Dreifachbestimmung ermittelt. Bei einer Abweichung der Messergebnisse für die gespikten Proben von den Messergebnissen für die ungespikte Probe von mehr als 10 % ist eine Interferenz gegeben, d. h. eine zuverlässige Faktor XIII-Bestimmung ist nicht möglich.

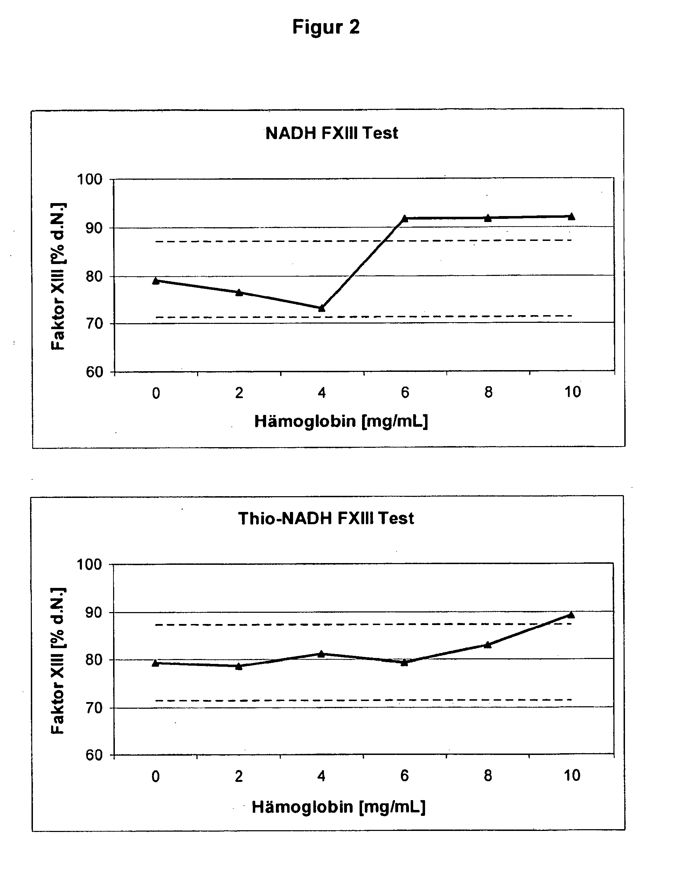

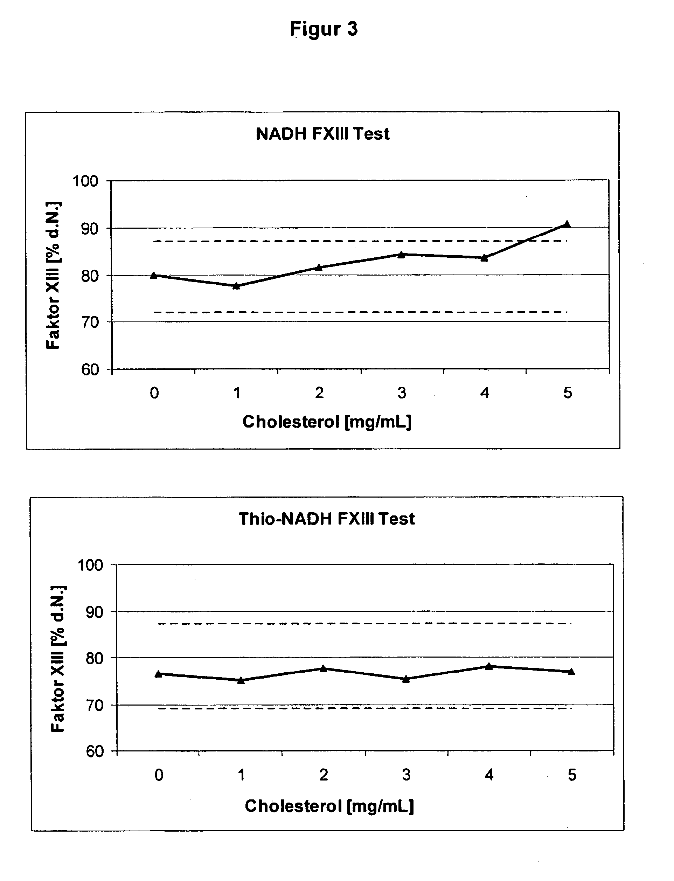

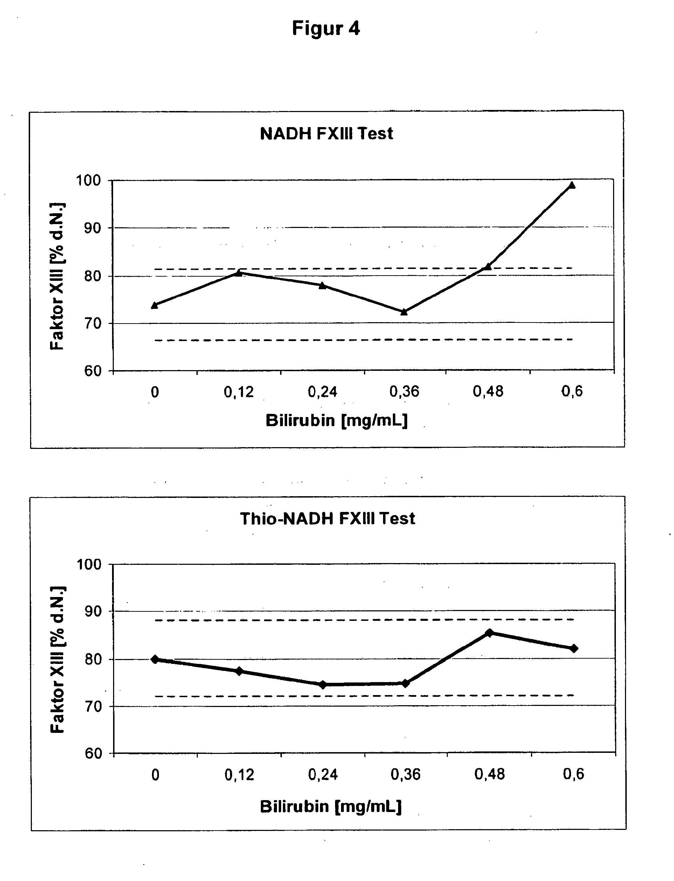

Die Ergebnisse für die Hämoglobin-, Cholesterol- und Bilirubin-gespikten Proben sind in den Figuren 2 bis 4 dargestellt.

In Tabelle 1 sind die Störsubstanzkonzentrationen angegeben, bis zu welchen keine Interferenz mit dem Berichrom® FXIII-Test (kurz: NADH F XIII-Test) bzw. mit dem erfindungsgemäßen Verfahren (kurz: Thio-NADH F XIII-Test) beobachtet wird. Der Vergleich der beiden Teste zeigt, dass der erfindungsgemäße Test höhere Störsubstanzkonzentrationen toleriert, also weniger störanfällig gegenüber probenintrinsischen Störsubstanzen ist. Bei den Störsubstanzen Cholesterol bzw. Bilirubin toleriert der erfindungsgemäße Thio-NADH Test sogar die höchste zudosierte Menge, so dass eine Interferenz vermutlich erst bei höheren Konzentrationen als in Tabelle 1 angegeben auftritt.

**Tabelle 1**

| | Keine Interferenz bis: | | |
|---|---|---|---|
| **F XIII-Test** | **Hämoglobin** | **Cholesterol** | **Bilirubin** |
| **NADH** | 4 mg/mL | 4 mg/mL | 0,36 mg/mL |
| **Thio-NADH** | 8 mg/mL | 5 mg/mL | 0,6 mg/mL |

## Patentansprüche

1. Verfahren zur Bestimmung von Faktor XIII in einer Probe, bei dem
a) die Probe mit einem oder mehreren Reagenzien enthaltend
I. eine Substanz oder ein Substanzgemisch zur Aktivierung des Faktor XIII zu Faktor XIIIa,
II. ein Akzeptorsubstrat für Faktor Xllla mit mindestens einer Glutaminylgruppe,
III. ein Aminogruppendonorsubstrat für Faktor XIIIa,
IV. ein NAD(P)H-Analogon mit einem Absorptionsmaximum, das oberhalb von 350 nm liegt, und
V. ein Mittel, das in Gegenwart von Ammoniak NAD(P)H zu NAD(P)+ oder ein NAD(P)H-Analoga zu dem entsprechenden NAD(P)+-Analoga zu oxidieren vermag,
vermischt wird und
b) die Änderung der Absorption des Testansatzes gemessen wird und wobei das NAD(P)H-Analogon Thio-NAD(P)H oder Seleno-NAD(P)H ist.

2. Verfahren gemäß Anspruch 1, wobei in Schritt a) die Probe weiterhin mit einem Fibrinaggregationshemmer vermischt wird.

3. Verfahren gemäß einem der vorhergehenden Ansprüche, wobei die Substanz zur Aktivierung des Faktor XIII zu Faktor Xllla Thrombin ist.

4. Verfahren gemäß einem der vorhergehenden Ansprüche, wobei das Akzeptorsubstrat für Faktor XIIIa mit mindestens einer Glutaminylgruppe ein Polypeptid ist, das mindestens einen Glutaminrest als Amin-Akzeptor aufweist.

5. Verfahren gemäß einem der vorhergehenden Ansprüche, wobei das Aminogruppendonorsubstrat für Faktor XIIIa ein primäres Amin ist, bevorzugterweise ein primäres Amin aus der Gruppe Ethanolamin, Putrescin, Cadaverin, Diaminoethan, Aminoethan, Glycinethylester und Glycinmethylester.

6. Verfahren gemäß einem der vorhergehenden Ansprüche, wobei das Mittel, das in Gegenwart von Ammoniak NAD(P)H zu NAD(P)+ oder ein NAD(P)H-Analoga zu dem entsprechenden NAD(P)+-Analoga zu oxidieren vermag, ein Enzym und ein Substrat für das Enzym umfasst.

7. Verfahren gemäß Anspruch 6, wobei das Enzym Glutamatdehydrogenase ist und das Substrat für das Enzym αKetoglutarat ist.

8. Verfahren gemäß einem der vorhergehenden Ansprüche, wobei in Schritt a) die Probe weiterhin mit einer Heparin-neutralisierenden Substanz, vorzugsweise mit Hexadimethrinbromid, und/oder mit Calciumchlorid vermischt wird.

9. Verfahren gemäß einem der vorhergehenden Ansprüche, wobei die Änderung der Absorption des Testansatzes mit Licht einer Wellenlänge von etwa 340 nm bis etwa 430 nm, bevorzugterweise mit Licht einer Wellenlänge von etwa 380 nm bis etwa 420 nm, ganz besonders bevorzugt mit Licht einer Wellenlänge von etwa 390 bis etwa 410 nm gemessen wird.

10. Verwendung des NAD(P)H-Analogons Thio-NAD(P)H in einem Verfahren zur Bestimmung von Faktor XIII in einer Probe.

11. Verwendung des NAD(P)H-Analogons Seleno-NAD(P)H in einem Verfahren zur Bestimmung von Faktor XIII in einer Probe.

12. Testkit zur Durchführung eines Verfahrens zur Bestimmung von Faktor XIII in einer Probe, wobei das Testkit folgende Komponenten umfasst:
a. ein erstes Reagenz enthaltend eine Substanz oder ein Substanzgemisch zur Aktivierung des Faktor XIII zu Faktor XIIIa, bevorzugterweise Thrombin;
b. ein zweites Reagenz enthaltend
• mindestens ein Akzeptorsubstrat für Faktor XIIIa mit mindestens einer Glutaminylgruppe,
• mindestens ein Aminogruppendonorsubstrat für Faktor XIIIa, bevorzugterweise ein primäres Amin, und
• mindestens ein Mittel, das in Gegenwart von Ammoniak NAD(P)H zu NAD(P)+ oder ein NAD(P)H-Analoga zu dem entsprechenden NAD(P)+-Analoga zu oxidieren vermag, wobei das Mittel bevorzugterweise aus Glutamatdehydrogenase und αKetoglutarat besteht; und
c. ein drittes Reagenz enthaltend mindestens ein NAD(P)H-Analogon mit einem Absorptionsmaximum, das oberhalb von 350 nm liegt und wobei das NAD(P)H-Analogon Thio-NAD(P)H oder Seleno-NAD(P)H ist.

13. Testkit gemäß Anspruch 12, wobei das erste Reagenz Thrombin zur Aktivierung des Faktor XIII zu Faktor XIIIa und zusätzlich Calciumchlorid und/oder einen Fibrinaggregationshemmer und/oder Hexadimethrinbromid enthält.

## Claims

1. Method for determining factor XIII in a sample where
a) the sample is mixed with one or more reagents comprising
I. a substance or a substance mixture for activating the factor XIII to factor XIIIa,
II. an acceptor substrate for factor XIIIa having at least one glutaminyl group,
III. an amino group donor substrate for factor XIIIa,
IV. an NAD(P)H analog having an absorption maximum above 350 nm, and
V. an agent which is capable of oxidizing NAD(P)H to NAD(P)+ or an NAD(P)H analog to the corresponding NAD(P)+ analog in the presence of ammonia,
and
b) the change in absorption of the test mixture is measured and where the NAD(P)H analog is thio-NAD(P)H or seleno-NAD(P)H.

2. Method according to Claim 1, where in step a) the sample is furthermore mixed with a fibrin aggregation inhibitor.

3. Method according to either of the preceding claims, where the substance for activating factor XIII to factor XIIIa is thrombin.

4. Method according to any of the preceding claims, where the acceptor substrate for factor XIIIa having at least one glutaminyl group is a polypeptide which has at least one glutamine residue as amine acceptor.

5. Method according to any of the preceding claims, where the amino group donor substrate for factor XIIIa is a primary amine, preferably a primary amine from the group consisting of ethanolamine, putrescine, cadaverine, diaminoethane, aminoethane, glycine ethyl ester and glycine methyl ester.

6. Method according to any of the preceding claims, where the agent capable of oxidizing, in the presence of ammonia, NAD(P)H to NAD(P)+ or an NAD(P)H analog to the corresponding NAD(P)+ analog comprises an enzyme and a substrate for the enzyme.

7. Method according to Claim 6, where the enzyme is glutamate dehydrogenase and the substrate for the enzyme is α-ketoglutarate.

8. Method according to any of the preceding claims, where in step a) the sample is furthermore mixed with a heparin-neutralizing substance, preferably with hexadimethrine bromide, and/or with calcium chloride.

9. Method according to any of the preceding claims, where the change in absorption of the test mixture is measured using light of a wavelength of about 340 nm to about 430 nm, preferably light of a wavelength of about 380 nm to about 420 nm, very particularly preferably light of a wavelength of about 390 to about 410 nm.

10. Use of the NAD(P)H analog thio-NAD(P)H in a method for determining factor XIII in a sample.

11. Use of the NAD(P)H analog seleno-NAD(P)H in a method for determining factor XIII in a sample.

12. Testkit for carrying out a method for determining factor XIII in a sample, where the test kit comprises the following components:
a. a first reagent comprising a substance or a substance mixture for activating factor XIII to factor XIIIa, preferably thrombin;
b. a second reagent comprising
• at least one acceptor substrate having at least one glutaminyl group for factor XIIIa,
• at least one amino group donor substrate for factor XIIIa, preferably a primary amine, and
• at least one agent capable of oxidizing, in the presence of ammonia, NAD(P)H to NAD(P) + or an NAD(P)H analog to the corresponding NAD(P)+ analog, the agent preferably consisting of glutamate dehydrogenase and α-ketoglutarate; and
c. a third reagent comprising at least one NAD(P)H analog having an absorption maximum above 350 nm and where the NAD(P)H analog is thio-NAD(P)H or seleno-NAD(P)H.

13. Testkit according to Claim 12, where the first reagent comprises thrombin for activating factor XIII to factor XIIIa and additionally calcium chloride and/or a fibrin aggregation inhibitor and/or hexadimethrine bromide.

## Revendications

1. Procédé de détermination du facteur XIII dans un échantillon, dans lequel
a) on mélange l'échantillon à un réactif ou à plusieurs réactifs contenant
I. une substance ou un mélange de substances d'activation du facteur XIII en le facteur XIIIa,
II. un substrat accepteur pour le facteur XIIIa ayant au moins un groupe glutaminyle,
III.un substrat donneur de groupes amino pour le facteur XIIIa,
IV. un analogue de NAD(P)H ayant un maximum d'absorption qui est au-dessus de 350 nm, et,
V. un agent qui, en présence d'ammoniaque, peut oxyder NAD(P)+ ou un analogue de NAD(P)H en l'analogue de NAD(P)+ correspondant
et
b) on mesure l'absorption de la masse de test et dans lequel l'analogue de NAD(P)H est le thio-NAD(P)H ou le sélénio-NAD(P)H.

2. Procédé suivant la revendication 1, dans lequel dans le stade a) on mélange l'échantillon en outre à un inhibiteur d'agrégation de la fibrine.

3. Procédé suivant l'une des revendications précédentes, dans lequel la substance d'activation du facteur XIII en le facteur XIIIa est la thrombine.

4. Procédé suivant l'une des revendications précédentes, dans lequel le substrat accepteur pour le facteur XIIIa ayant au moins un groupe glutaminyle est un polypeptide qui a au moins un reste glutamine comme accepteur amine.

5. Procédé suivant l'une des revendications précédentes, dans lequel le substrat donneur de groupes amino pour le facteur XIIIa est une amine primaire, de préférence une amine primaire choisie dans le groupe éthanolamine, putrescine, cadavérine, diaminoéthane, aminoéthane, ester éthylique de glycine et ester méthylique de glycine.

6. Procédé suivant l'une des revendications précédentes, dans lequel l'agent, qui en présence d'ammoniaque permet d'oxyder NAD(P)H en NAD(P)+ ou un analogue de NAD(P)H en l'analogue de NAD(P)+ correspondant, comprend un enzyme et un substrat pour l'enzyme.

7. Procédé suivant la revendication 6, dans lequel l'enzyme est la glutamate déshydrogénase et le substrat pour l'enzyme est l'αcétoglutarate.

8. Procédé suivant l'une des revendications précédentes, dans lequel dans le stade a) on mélange l'échantillon en outre à une substance neutralisant l'héparine, de préférence à du bromure d'hexadiméthrine et/ou à du chlorure de calcium.

9. Procédé suivant l'une des revendications précédentes, dans lequel on mesure la variation de l'absorption de la masse de test par de la lumière d'une longueur d'onde d'environ 340 nm à environ 430 nm, de préférence par de la lumière d'une longueur d'onde d'environ 380 nm à environ 420 nm, d'une manière tout à fait préférée par de la lumière d'une longueur d'onde d'environ 390 à environ 410 nm.

10. Utilisation de l'analogue de NAD(P)H thio-NAD(P)H dans un procédé de détermination du facteur XIII dans un échantillon.

11. Utilisation de l'analogue de NAD(P)H sélénio-NAD(P)H dans un procédé de détermination du facteur XIII dans un échantillon.

12. Trousse de test pour effectuer un procédé de détermination du facteur XIII dans un échantillon, la trousse de test comprenant les constituants suivants :
a.un premier réactif contenant une substance ou un mélange de substances d'activation du Facteur XIII en le facteur XIIIa, d'une manière préférée de la thrombine ;
b. un deuxième réactif contenant
• au moins un substrat accepteur pour le facteur XIIIa ayant au moins un groupe glutaminyle,
• au moins un substrat donneur de groupes amino pour le facteur XIIIa, d'une manière préférée une amine primaire, et
• au moins un agent qui, en présence d'ammoniaque permet d'oxyder du NAD(P)H en du NAD(P)+ ou un analogue de NAD(P)H en l'analogue de NAD(P)+ correspondant, l'agent étant de préférence composé de glutamate déshydrogénase et d'αcétoglutarate ; et
c.un troisième réactif contenant au moins un analogue de NAD(P)H ayant un maximum d'absorption, qui est au-dessus de 350 nm et dans lequel l'analogue de NAD(P)H est le thio-NAD(P)H ou le sélénio-NAD(P)H.

13. Trousse de tests suivant la revendication 12, dans laquelle le premier réactif contient de la thrombine d'activation du facteur XIII en le facteur XIIIa et en outre du chlorure de calcium et/ou un inhibiteur d'agrégation de la fibrine et/ou du bromure d'hexadiméthrine.
